# EUROPEAN PATENT SPECIFICATION

(11) **EP 4 248 887 B1**
(45) Date of publication and mention of the grant of the patent: **15.07.2026**
(21) Application number: 23184087.7
(22) Date of filing: 11.11.2019
(51) Int. Cl.: A61B 17/17

(54) **DRILL GUIDE**
BOHRFÜHRUNG
GUIDE DE FORAGE

(30) Priority: 16.11.2018 US 201862768299 P
(43) Date of publication of application: 27.09.2023
(62) Divisional of application: 19817457.5
(73) Proprietor: ConMed Corporation, Largo, FL 33773 (US)
(72) Inventor: THIBODEAU, Robert A., Saint Petersburg, 33710 (US); SUMMITT, Matthew C., Palm Harbor, 34683 (US); ALFONSO, Gregory A., Seffner, 33584 (US); CARSON, Christopher, Seymour, 06483 (US)
(74) Representative: Strehl & Partner mbB

(56) References cited:
- US-A- 4 722 331
- US-A- 6 120 511
- US-A1- 2012 197 259
- US-A1- 2016 302 804
- No Search

## Description

### CROSS-REFERENCE TO RELATED APPLICATIONS

This application claims priority to U.S. Provisional Patent Application Serial No. 62/768,299, filed on November 16, 2018 and entitled "Foot & Ankle - Hand & Wrist Drill Guide."

### BACKGROUND OF THE INVENTION

### 1. Field of the Invention

The present invention is directed generally to surgical tools and instruments and, more particularly, to a multi-axis drill guide assembly for precise drilling and guidewire placement.

### 2. Description of Related Art

During orthopedic surgeries, holes are often drilled in bones. In some procedures, a drill guide is used to line-up the drill to ensure the drilled tunnel is in the appropriate location. Many bone surfaces to be drilled have severe contours that vary from patient to patient. Due to the contours, it can be difficult to properly seat the drill guide against the bone. This is particularly true when the drill guide is used to assist in drilling a tibial tunnel during ACL reconstruction.

Many conventional drill guide assemblies are used in conjunction with a guide arm and a drill guide sleeve. Conventional drill guide assemblies also require a threaded mechanism to prevent the guide arm from falling out of the drill guide assembly. Further, many traditional drill guide sleeves have a fixed geometry. These sleeves are malleted into place into the bone, which can cause misalignment.

Therefore, there is a need for a drill guide with more than one type of feature for fixing the drill guide in place relative to the bone and a drill guide arm that provides a wide range of angles for drill placement.

US4722331A discloses a surgical drill guide device, comprising: a housing having a sleeve disposed through an aperture extending through a proximal portion thereof for maintaining slideable communication with an insertion member, the insertion member being elongated and having an aimer tip and an insertion knob for disposing the insertion member slideably through the housing along an insertion axis; and an aimer arm engaging a slot in a distal portion of the housing for arcuate movement thereto, the aimer arm having a proximate end engaging the slot and an opposed distal end; an aimer guide coupled to the distal end of the aimer arm, the aimer arm extending in an intersecting manner with the insertion axis; and a template attached to the annex guide, the template having an aperture defining a drilling footprint, the drilling footprint located on the insertion axis and corresponding to a bone tunnel through which a surgical attachment is passed; wherein the template has a flattened construction providing a low profile, the flattened construction facilitating insertion between surgical members for disposing the template in parallel alignment with a surgical landmark for defining a location of the bone tunnel; and wherein the proximal portion of the housing retains the insertion member coplanar with the aimer arm.

Description of the Related Art Section Disclaimer: To the extent that specific patents/publications/products are discussed above in this Description of the Related Art Section or elsewhere in this disclosure, these discussions should not be taken as an admission that the discussed patents/publications/products are prior art for patent law purposes. For example, some or all of the discussed patents/publications/products may not be sufficiently early in time, may not reflect subject matter developed early enough in time and/or may not be sufficiently enabling so as to amount to prior art for patent law purposes.

### SUMMARY OF THE INVENTION

The drill guide assembly of the present invention is defined in Claim 1. Further advantageous features are set out in the dependent claims.

Embodiments of the present invention are directed to a multi-axis drill guide assembly for precise drilling and guidewire placement. According to one aspect, the drill guide assembly includes a guide arm having a first end and a second end with a curved slot extending from the first end toward the second end. The drill guide assembly also includes a curved guide extension arm having a first end and a second end. The first end of the curved guide extension arm is slidable within the curved slot of the guide arm. The drill guide assembly additionally has a probe arm rotatably connected to the second end of the guide arm. The probe arm is rotatable to a position that is non-planar relative to the guide arm.

According to another aspect, the drill guide assembly includes a guide arm having a first end and a second end with a curved slot extending from the first end toward the second end. The drill guide assembly also includes a curved guide extension arm having a first end and a second end. The first end of the curved guide extension arm is slidable within the curved slot of the guide arm. The drill guide assembly additionally has a drill guide attached to the second end of the curved guide extension arm. The drill guide extends along a first axis. The drill guide assembly also includes a probe arm having a first end and a second end. The first end of the probe arm is rotatably connected to the second end of the guide arm. The probe arm is rotatable toward the curved guide extension arm. A probe is attached to the second end of the probe arm and the probe extends along a second axis. In a first configuration, the first axis is at a first angle relative to the second axis and in a second configuration, the first axis is at a second angle relative to the second axis. The first angle is different from the second angle.

According to yet another aspect, the drill guide assembly includes a guide arm having a first end and a second end with a curved slot extending from the first end toward the second end. The drill guide assembly also includes a curved guide extension arm having a first end and a second end. The first end of the curved guide extension arm is slidable within the curved slot of the guide arm. The drill guide assembly additionally has a probe arm having a first end and a second end. The first end of the probe arm is rotatably connected to the second end of the guide arm. A tapered probe is attached to the second end of the probe arm.

These and other aspects of the invention will be apparent from and elucidated with reference to the embodiment(s) described hereinafter.

### BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:
FIG. 1 is a side perspective view schematic representation of a drill guide assembly, according to an embodiment;
FIG. 2 is a perspective view schematic representation of the probe of the probe assembly, according to an embodiment;
FIG. 3 is a top perspective view schematic representation of the drill guide assembly, according to an embodiment;
FIG. 4 is a side perspective view schematic representation of the pivot locking screw, according to an embodiment;
FIG. 5 is a side perspective view schematic representation of the drill guide assembly in a first configuration, according to an embodiment;
FIG. 6 is a side perspective view schematic representation of the drill guide assembly in a second configuration, according to an embodiment; and
FIG. 7 is a close-up perspective view schematic representation of the drill guide, according to an embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the spirit and/or scope of the underlying inventive concepts will be apparent to those skilled in the art from this disclosure.

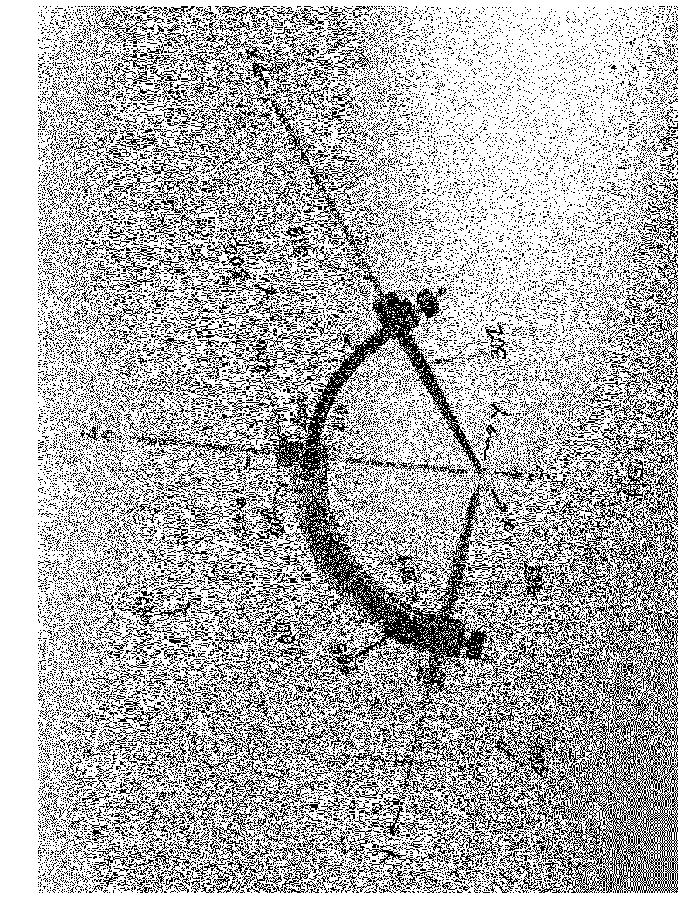

Referring now to the figures, wherein like reference numerals refer to like parts throughout, FIG. 1 shows a side perspective view schematic representation of a drill guide assembly 100, according to an embodiment. The drill guide assembly 100 comprises a curved or bent guide arm 200. The guide arm 200 has a first end 202 connected to a probe assembly 300 and a second end 204 connected to a drill guide sub-assembly 400. The probe assembly 300 comprises a probe 302 extending along a longitudinal first axis x - x. A drill guide 408 is connected to the drill guide sub-assembly 400 and extends along a second axis y - y, which is transverse relative to the first axis x - x in the depicted embodiment.

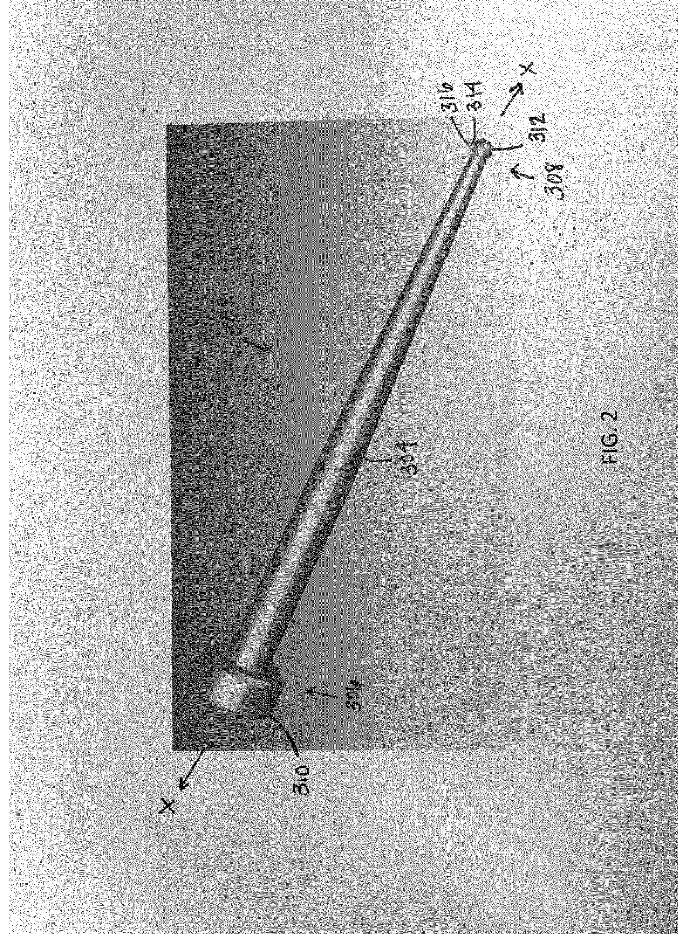

Turning now to FIG. 2, there is shown a perspective view schematic representation of the probe 302 of the probe assembly 300, according to an embodiment. The probe 302 comprises an elongated shaft 304 with a first end 306 and a second end 308. In the depicted embodiment, the probe 302 is tapered, decreasing in diameter from the first end 306 to the second end 308. The first end 306 of the probe 302 comprises a knurled cylindrical cap 310 for maintaining positioning of the probe 302, as described in detail below. The second end 308 of the probe 302 is rounded. In the depicted embodiment, the second end 308 of the probe 302 is spherical. A rounded or spherical second end 308 of the probe 302 protects any articular surfaces while advancing to the desired location in the joint.

Still referring to FIG. 2, the rounded or spherical second end 308 comprises an outer surface 312 with a circumference. A spike 314 extends from the outer surface 312 of the rounded or spherical second end 308. As shown in FIG. 2, the spike 314 extends to a point 316. The spike 314 extends at angle relative to the first axis x - x (through the probe 302). In the depicted embodiment, the spike 314 is approximately or equal to 90 degrees (i.e., substantially perpendicular) relative to the first axis x - x. The spike 314 is used to grip the bone while also providing a target for a drill pin location, as discussed below. In an embodiment, the probe 302 is cannulated such that the first end 306 and the second end 308 are open, allowing a first guidewire 318 (FIG. 1) to pass through the probe 302 to further secure the position of the drill guide 408.

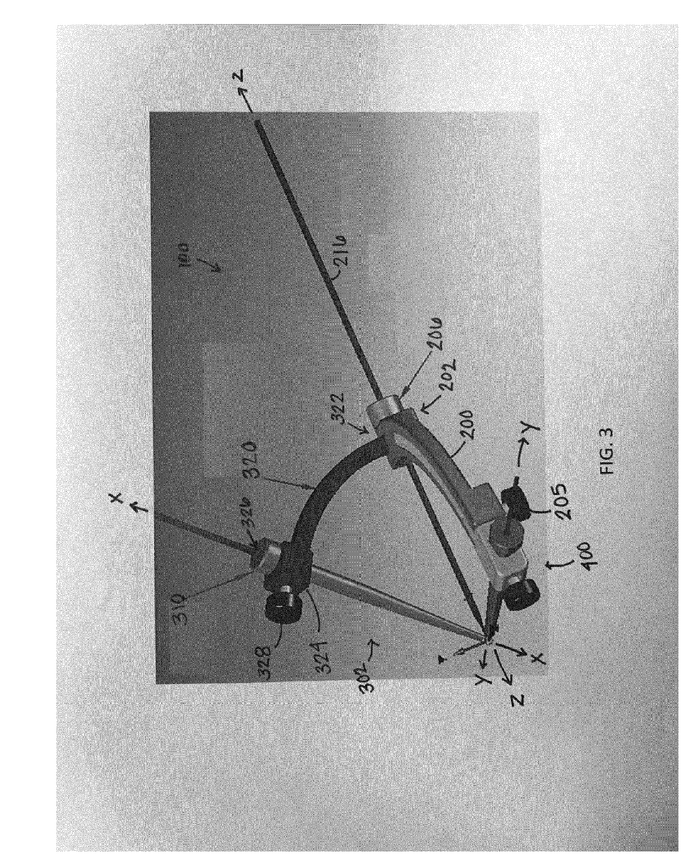

Referring to FIG. 3, there is shown a top perspective view schematic representation of the drill guide assembly 100, according to an embodiment. The probe 302 is connected to the guide arm 200 via a probe arm 320. In the depicted embodiment, the probe arm 320 is curved or bent in a fashion similar to the guide arm 200. The probe arm 320 comprises a first end 322 and a second end 324. The first end 322 connects to the guide arm 200, as described in detail below. The second end 324 connects to the probe 302.

The second end 324 of the probe arm 320 comprises a channel 326 extending therethrough along the first axis x - x. The channel 326 is sized and configured to accommodate movement of the probe 302 in and out of the channel 326. The cap 310 of the probe 302 functions as a stop and prevents the probe 302 from sliding entirely through the channel 326. In the depicted embodiment, the second end 324 of the probe arm 320 is cubic or otherwise rectangular. Although the second end 324 may have other geometries, the cubic or rectangular shape of the second end 324 accommodates a locking mechanism 328.

In the embodiment shown in FIG. 3, the locking mechanism 328 is a locking screw. When the probe 302 is in the desired position (i.e., at a particular depth within the channel 326), the locking screw 328 is extended into the channel 326 in the second end 324. In the depicted embodiment, the locking screw 328 is transverse to the first axis x - x. Specifically, the locking screw 328 extends in a direction or along an axis that is substantially perpendicular to the first axis x - x. To secure the probe 302 in place within the channel 326, the locking screw 328 is tightened against the probe 302 within the channel 326.

As mentioned above, the first end 322 of the probe arm 320 is connected to the guide arm 200. In particular, the first end 322 of the probe arm 320 is rotatably attached to the guide arm 200 via a pivotable connector 206. The pivotable connector 206 is a pivot locking screw in the embodiment shown in FIG. 3. The pivot locking screw 206 extends into the first end 202 of the guide arm 200, as shown in FIG. 1. The probe arm 320 is preferably rotatable up to a total of 240 degrees about the pivot locking screw 206. However, in accordance with an alternative embodiment, probe arm 320 is rotatable more than a total of 240 degrees about the pivot locking screw 206 (e.g., up to and beyond 360 degrees in certain embodiments), as should be understood by a person of ordinary skill in the art in conjunction with a review of this disclosure.

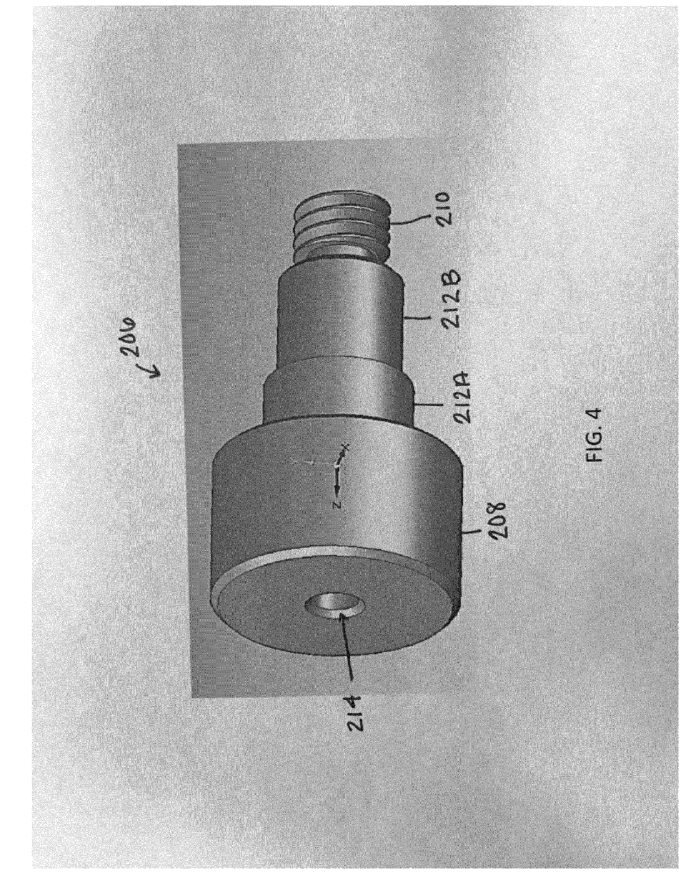

Turning now to FIG. 4, there is shown a side perspective view schematic representation of the pivot locking screw 206, according to an embodiment. The pivot locking screw 206 comprises a first cylindrical portion 208 (with a first diameter) connected to threads 210, with one or more additional cylindrical portions 212A, 212B attached therebetween. In the depicted embodiment, the pivot locking screw 206 has the first cylindrical portion 208 with a first diameter connected to a second cylindrical portion 212A with a second diameter, the second cylindrical portion 212A connected to a third cylindrical portion 212B with a third diameter, and the third cylindrical portion 212B connected to the threads 210. In an embodiment, the first diameter is larger than the second diameter, the second diameter is larger than the third diameter, and the threads 210 have a diameter that is smaller than the third diameter.

In use, the first end 322 of the probe arm 320 extends around at least one of the additional cylindrical portions 212A, 212B, while the threads 210 are screwed into the guide arm 200 and a portion of the guide arm 200 extends around the first cylindrical portions 208 (FIG. 1). In the embodiment shown in FIG. 4, the pivot locking screw 206 is cannulated, providing a channel 214 extending through the pivot locking screw 206, through the first cylindrical portion 308, additional cylindrical portions 212A, 212B, and the threads 210 for placement of a second guidewire 216 (FIGs. 1 and 3). The second guidewire 216 extends along a third axis z - z, which is different than the first axis x - x and second axis y - y.

Still referring to FIG. 4, the pivot locking screw 206 is used to join the probe arm 320 and the guide arm 200. The pivot locking screw 206 can also be used to lock the probe arm 320 in position after the desired angle has been achieved. As stated above, the pivot locking screw 206 is passed through the first end 202 of the guide arm 200 and then through the probe arm 320. The pivot locking screw 206 is secured by screwing the threads 210 into the first end 202 of the drill guide arm 200. The locking action is achieved by tightening the pivot locking screw 206 in a clockwise direction, causing the guide arm 200 to compress the probe arm 320.

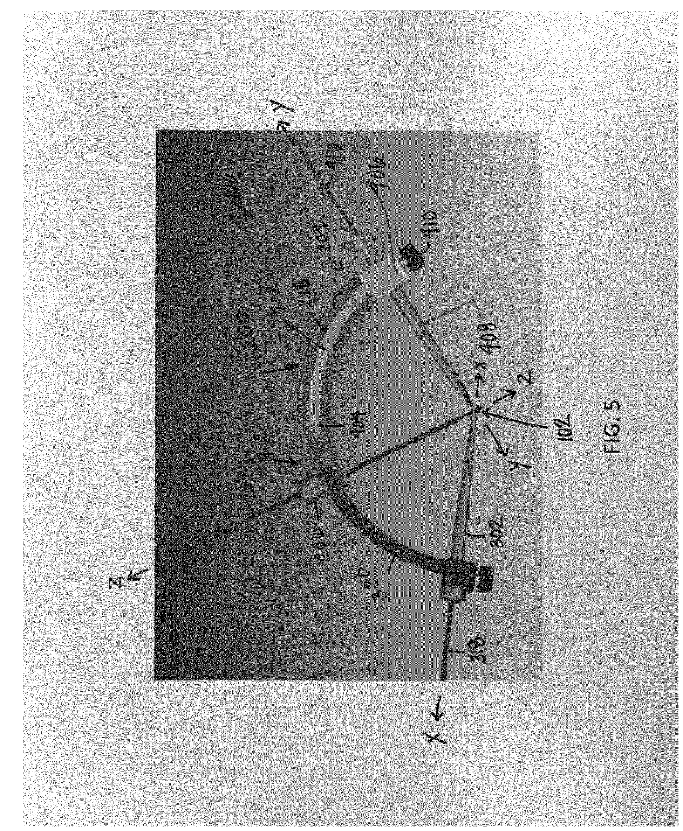

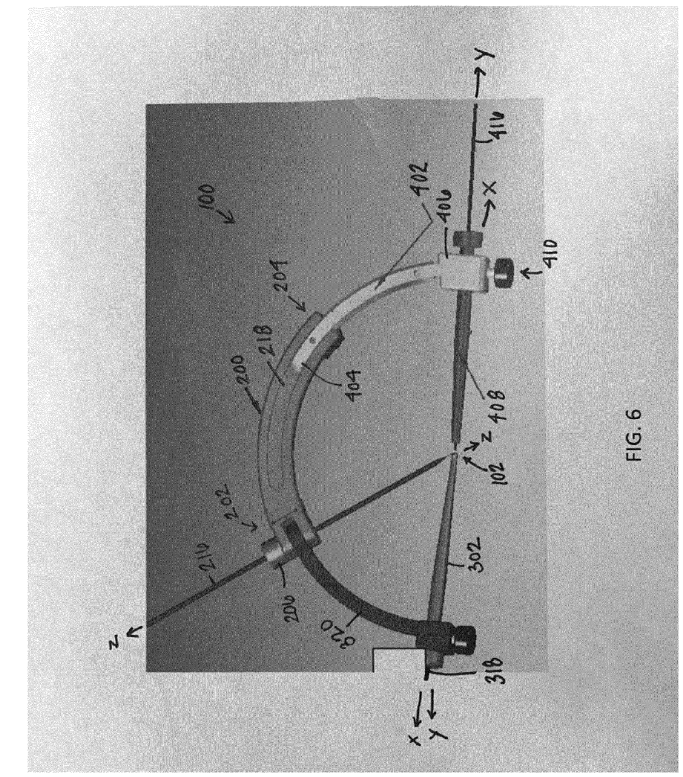

FIGs. 5 and 6 show side perspective views schematic representations of the drill guide assembly 100 in a first configuration and a second configuration, respectively. The guide arm 200 comprises a curved or bent slot 218 extending along the length of the guide arm 200. In particular, the slot 218 begins at the second end 204 of the guide arm 200 and extends toward the first end 202 of the guide arm 200. The curved or bent slot 218 is sized and configured to slidably receive a drill guide extension arm 402 of the drill guide sub-assembly 400. Accordingly, the drill guide extension arm 402 is curved or bent to fit within the slot 218 in the drill guide arm 200. At its first end 404, the drill guide extension arm 402 is free to slide within the slot 218 in the drill guide arm 200, while its second end 406 is connected to the drill guide 408.

The guide arm 200 comprises a connector 205 (e.g., a lock screw) to secure or fix the drill guide extension arm 402 in a position within the slot 218. As shown in FIGs. 1 and 3, the connector 205 extends into the second end 204 of the guide arm 200. The connector 205 locks the drill guide 408 at an angle relative to the second guidewire 216. In other words, the connector 205 locks the first axis x - x at an angle relative to the third axis z - z.

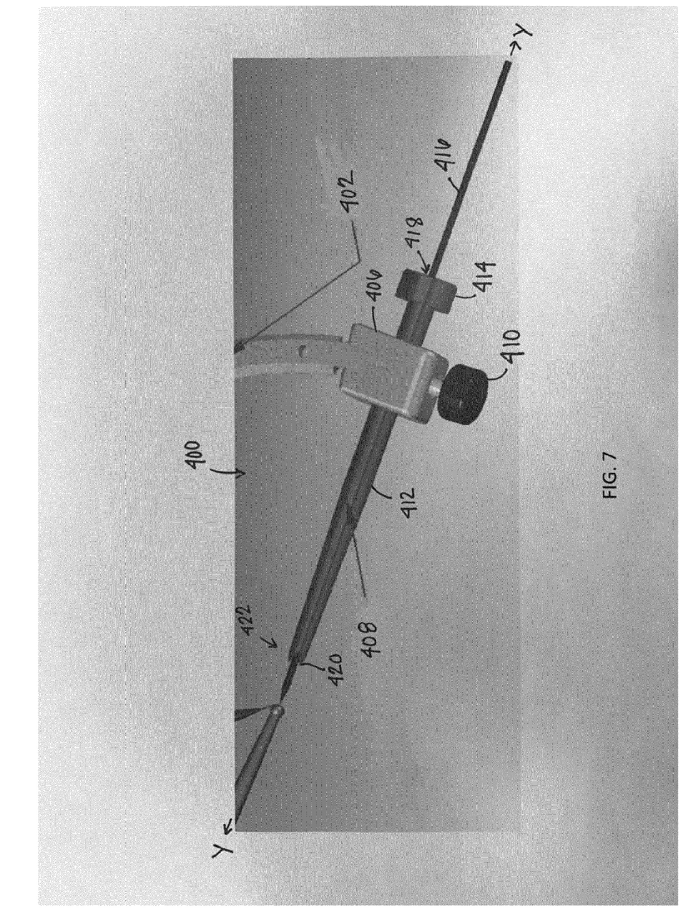

Turning briefly to FIG. 7, there is shown a close-up perspective view schematic representation of the drill guide 408, according to an embodiment. In the depicted embodiment, the second end 406 of the drill guide extension arm 402 is cubic or otherwise rectangular to accommodate a connector 410. In the depicted embodiment, the connector 410 is a lock screw that extends into the second end 406 of the drill guide extension arm 402 at an angle relative to the second axis y - y. Specifically, the lock screw 410 extends in a direction or along an axis that is substantially perpendicular to the second axis y - y.

Still referring to FIG. 7, the drill guide 408 has a tapered elongated shaft 412 connected to a cylindrical cap 414. In an embodiment, the drill guide 408 is cannulated such that a drill pin 416 can be inserted through a channel 418 in the cap 414 and the elongated shaft 412. In one example embodiment, the drill guide 408 is cannulated to accept a .072 diameter drill pin 416 (but other sized drill pins can be used, as should be understood and appreciated by a person of ordinary skill in the art in conjunction with a review of this disclosure). The cap 414 has an external diameter larger than an internal diameter of the channel 418 such that the cap 414 prevents the drill guide 408 from sliding entirely through the channel 418 and into the surgical site.

As also shown in FIG. 7, the elongated shaft 412 of the drill guide 408 comprises a crown 420 at a free end 422. Generally, the crown 420 is one or more jagged or otherwise sharp edges at the free end 422 of the drill guide 408. In use, the probe 302 is first placed in the desired location and then the drill guide 408 is advanced until the crown 420 has made contact with the bone. Thereafter, the lock screw 410 is then tightened to secure the drill guide 408 in place.

Referring back to FIG. 5, in the first configuration, the drill guide extension arm 402 is at a first position within the slot 218 in the guide arm 200. When the drill guide extension arm 402 is in the first position, the drill guide 408 is at a first angle relative to the probe 302. Stated differently, the first axis x - x is at a first angle relative to the second axis y - y. To move to the second configuration, shown in FIG. 6, the drill guide extension arm 402 is moved (at least partially) within the slot 218 to a second position. In the second position, the drill guide 408 is at a second angle relative to the probe 302. As follows, the first axis x - x is at a second angle relative to the second axis y - y. The first angle is different than the second angle. In the depicted embodiment, the second angle is greater than the first angle. In some instances, the first axis x - x is transverse with respect to the second axis y - y, although it is possible that the first axis x - x and the second axis y - y are the same axis.

Still referring to FIGs. 5 and 6, in both the first and second configurations, the probe 302 and the drill guide 408 meet at or extend toward a drill space 102. In the depicted embodiment, the second guidewire 216 extends through the pivot locking screw 206 and also meets at or extends toward the drill space 102. The first guidewire 318 extending through the probe 302 also meets at or extends toward the drill space 102. The first and second guidewires 216, 318 are optionally used to further secure the position of the drill guide 408. The movement described above with reference to FIGs. 5 and 6 is movement of the drill guide 408 within one plane to achieve a first configuration and second configuration. A third configuration can be achieved by rotating the probe arm 320 about the pivot locking screw 206 toward the drill guide sub-assembly 400, as shown in FIG. 3.

In FIGs. 5 and 6, the drill guide assembly 100 is substantially planar, meaning that the drill guide arm 200 extends within approximately the same plane as the drill guide 408 (and drill guide extension arm 402) and the probe 302 (and the probe arm 320). In the third configuration, the probe arm 320 is rotated about the pivot locking screw 206 and the third axis z - z such that the probe 302 and the probe arm 320 are no longer substantially planar with the drill guide arm 200 and the drill guide 408 (and drill guide extension arm 402), as shown in FIG. 3. However, despite rotating the probe 302 and probe arm 320 into another plane, the probe 302 still meets at or extends to the drill space 102 with the drill guide 408. In the embodiment shown in FIG. 3, the second guidewire 318 extends through the pivot locking screw 206 and also meets at or extends to the drill space 102 with the probe 302 and the drill guide 408. Thus, in the first, second, and third configurations, the probe 302 (and the first guidewire 318) and the drill guide 408 (and the drill pin 416), and in some instances, the second guidewire 216, all converge at the drill space 102.

All definitions, as defined and used herein, should be understood to control over dictionary definitions, definitions in documents mentioned herein, and/or ordinary meanings of the defined terms.

While various embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims, embodiments may be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a method or device that "comprises", "has", "includes" or "contains" one or more steps or elements. Likewise, a step of method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The corresponding structures, materials, acts and equivalents of all means or step plus function elements in the claims below, if any, are intended to include any structure, material or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

The present invention further embraces the following aspects. According to a first aspect, a drill guide assembly comprises: a guide arm having a first end and a second end with a curved slot extending from the first end toward the second end; a curved guide extension arm comprising a first end and a second end, the first end slidable within the curved slot of the guide arm; a probe arm rotatably connected to the second end of the guide arm, wherein the probe arm is rotatable to a position that is non-planar relative to the guide arm. The probe arm may be rotatably connected to the second end of the guide arm via a pivot locking screw which may further be cannulated and configured to receive a guidewire therethrough.

In the drill guide assembly of the first aspect, the probe arm may be rotatable up to and including at least 240 degrees. The drill guide assembly of the first aspect may further comprise a drill guide extending through the second end of the curved guide extension arm and my further comprise a probe extending through the probe arm. The probe and the drill guide may then converge at a drill space.

A drill guide assembly according to a second aspect comprises: a guide arm having a first end and a second end and a curved slot extending from the first end toward the second end; a curved guide extension arm having a first end and a second end, the first end slidable within the curved slot of the guide arm; a drill guide attached to the second end of the curved guide extension arm, the drill guide extending along a first axis; a probe arm having a first end and a second end, the first end rotatably connected to the second end of the guide arm; wherein the probe arm is rotatable toward the curved guide extension arm; and a probe attached to the second end of the probe arm, the probe extending along a second axis; wherein in a first configuration, the first axis is at a first angle relative to the second axis and in a second configuration, the first axis is at a second angle relative to the second axis; and wherein the first angle is different from the second angle.

In the drill guide assembly according to the second aspect, the curved guide extension arm may in the first configuration be at a first position within the curved slot and the curved guide extension arm may in the second configuration be at a second position within the curved slot, and the first and second positions are different. In the first configuration, the first axis may also be transverse with respect to the second axis. In the first configuration and the second configuration, the probe and the drill guide may also converge at a drill space. The drill guide may comprise a distal end having a crown or may be cannulated and configured to receive a drill pin therethrough. Also the probe may be cannulated and configured to receive a guidewire therethrough.

A drill guide assembly according to a third aspect comprises: a guide arm having a first end and a second end with a curved slot extending from the first end toward the second end; a curved guide extension arm having a first end and a second end, the first end slidable within the curved slot of the guide arm; a probe arm having a first end and a second end, the first end rotatably connected to the second end of the guide arm; and a tapered probe attached to the second end of the probe arm.

In the drill guide assembly according to the third aspect, the tapered probe may have a first end and a second end, decreasing in diameter from the first end toward the second end. The drill guide assembly may further comprise a cap at the first end of the tapered probe. The second end of the tapered probe may be spherical. The drill guide assembly may further comprise a spike extending from an outer surface of the spherical second end of the tapered probe, wherein the spike may further be substantially perpendicular to a longitudinal axis extending through the probe.

## Claims

1. A drill guide assembly (100), comprising:
a guide arm (200) having a first end (202) and a second end (204) with a curved slot (218) extending from the first end toward the second end;
a curved guide extension arm (402) comprising a first end (404) and a second end (406), the first end being slidable within the curved slot of the guide arm; and
a probe arm (320) having a first end (322) and a second end (324), the first end of the probe arm rotatably connected to the first end of the guide arm via a pivot locking screw (206), wherein:
the pivot locking screw is configured to allow the probe arm to rotate to a desired position that is non-planar relative to the guide arm and to lock the probe arm in the desired position, thereby preventing it from further rotating with respect to the guide arm, and
the pivot locking screw is cannulated and configured to receive a guidewire (216) therethrough.

2. The drill guide assembly of claim 1, wherein the pivot locking screw (206) comprises:
a first cylindrical portion (208) connected to a threaded portion (210), and
a second cylindrical portion (212A) positioned between the first cylindrical portion and the threaded portion.

3. The drill guide assembly of claim 2,
wherein the first cylindrical portion (208) has a first diameter and the second cylindrical portion (212A) has a second diameter wherein the first diameter is larger than the second diameter, and
wherein the threaded portion (210) has a third diameter wherein the third diameter is smaller than the second diameter.

4. The drill guide assembly of claim 3,
wherein the first end (202) of the guide arm (200) comprises a forked end with a first forked end arm and a second forked end arm forming a first space therebetween,
wherein the first end (322) of the probe arm (320) is positioned within the first space, and
wherein the pivot locking screw (206) is positioned through the first forked end arm, the first space and the second forked end arm, and wherein the pivot locking screw is configured to compress the probe arm positioned within the first space and between the first forked end arm and the second forked end arm.

5. The drill guide assembly of claim 1, further comprising a drill guide (408) extending through the second end (406) of the curved guide extension arm (402).

6. The drill guide assembly of claim 5, further comprising a probe (302) attached to the second end (324) of the probe arm (320);
wherein the probe is cannulated and configured to receive a guidewire (318) therethrough.

7. The drill guide assembly of claim 1 or 2, wherein the probe arm (320) is rotatable up to and including at least 240 degrees.

8. The drill guide assembly of claim 7, wherein the probe (302) extends through the probe arm (320).

9. The drill guide assembly of claim 8, wherein the probe and the drill guide (408) converge at a drill space (102).

## Patentansprüche

1. Bohrführungsanordnung (100), umfassend:
einen Führungsarm (200) mit einem ersten Ende (202) und einem zweiten Ende (204) mit einem gebogenen Schlitz (218), der vom ersten Ende zum zweiten Ende verläuft;
einen gebogenen Führungsverlängerungsarm (402) mit einem ersten Ende (404) und einem zweiten Ende (406), wobei das erste Ende in dem gebogenen Schlitz des Führungsarms gleiten kann; und
einen Sondenarm (320) mit einem ersten Ende (322) und einem zweiten Ende (324), wobei das erste Ende des Sondenarms über eine Schwenkverriegelungsschraube (206) drehbar mit dem ersten Ende des Führungsarms verbunden ist, wobei:
die Schwenkverriegelungsschraube so eingerichtet ist, dass sie es dem Sondenarm ermöglicht, in eine gewünschte Position zu drehen, die nicht-planar relativ zu dem Führungsarm ist, und den Sondenarm in der gewünschten Position zu verriegeln, wodurch verhindert wird, dass er sich weiter bezüglich des Führungsarms dreht, und
die Schwenkverriegelungsschraube kanüliert und so eingerichtet ist, dass sie einen Führungsdraht (216) durch sich hindurch aufnimmt.

2. Bohrführungsanordnung nach Anspruch 1, wobei die Schwenkverriegelungsschraube (206) umfasst:
einen ersten zylindrischen Abschnitt (208), der mit einem Gewindeabschnitt (210) verbunden ist, und
einen zweiten zylindrischen Abschnitt (212A), der zwischen dem ersten zylindrischen Abschnitt und dem Gewindeabschnitt positioniert ist.

3. Bohrführungsanordnung nach Anspruch 2,
wobei der erste zylindrische Abschnitt (208) einen ersten Durchmesser aufweist und der zweite zylindrische Abschnitt (212A) einen zweiten Durchmesser aufweist, wobei der erste Durchmesser größer als der zweite Durchmesser ist, und
wobei der Gewindeabschnitt (210) einen dritten Durchmesser aufweist, wobei der dritte Durchmesser kleiner als der zweite Durchmesser ist.

4. Bohrführungsanordnung nach Anspruch 3,
wobei das erste Ende (202) des Führungsarms (200) ein gegabeltes Ende mit einem ersten gegabelten Endarm und einem zweiten gegabelten Endarm umfasst, die einen ersten Raum dazwischen bilden,
wobei das erste Ende (322) des Sondenarms (320) innerhalb des ersten Raums positioniert ist, und
wobei die Schwenk-Verriegelungsschraube (206) durch den ersten gegabelten Endarm, den ersten Raum und den zweiten gegabelten Endarm positioniert ist, und wobei die Schwenkverriegelungsschraube dazu eingerichtet ist, den Sondenarm zu komprimieren, der innerhalb des ersten Raums und zwischen dem ersten gegabelten Endarm und dem zweiten gegabelten Endarm positioniert ist.

5. Bohrführungsanordnung nach Anspruch 1, ferner umfassend eine Bohrführung (408), die durch das zweite Ende (406) des gebogenen Führungsverlängerungsarms (402) verläuft.

6. Bohrführungsanordnung nach Anspruch 5, ferner umfassend eine Sonde (302), die am zweiten Ende (324) des Sondenarms (320) angebracht ist;
wobei die Sonde kanüliert und so eingerichtet ist, dass sie einen Führungsdraht (318) durch sich hindurch aufnimmt.

7. Bohrführungsanordnung nach Anspruch 1 oder 2, wobei der Sondenarm (320) bis zu und einschließlich mindestens 240 Grad drehbar ist.

8. Bohrführungsanordnung nach Anspruch 7, wobei die Sonde (302) durch den Sondenarm (320) verläuft.

9. Bohrführungsanordnung nach Anspruch 8, wobei die Sonde und die Bohrführung (408) an einem Bohrraum (102) konvergieren.

## Revendications

1. Ensemble (100) de guide-foret, comprenant :
un bras (200) de guidage ayant une première extrémité (202) et une deuxième extrémité (204) avec une fente incurvée (218) s'étendant depuis la première extrémité vers la deuxième extrémité ;
un bras incurvé (402) d'extension de guidage comprenant une première extrémité (404) et une deuxième extrémité (406), la première extrémité étant coulissante à l'intérieur de la fente incurvée du bras de guidage ; et
un bras (320) de sonde ayant une première extrémité (322) et une deuxième extrémité (324), la première extrémité du bras de sonde connectée rotative à la première extrémité du bras de guidage par l'intermédiaire d'une vis (206) de blocage de pivot, dans lequel :
la vis de blocage de pivot est configurée pour permettre au bras de sonde de tourner jusqu'à une position désirée qui est non plane par rapport au bras de guidage et pour bloquer le bras de sonde dans la position désirée, l'empêchant ainsi de continuer à tourner par rapport au bras de guidage, et
la vis de blocage de pivot est canulée et configurée pour recevoir un fil (216) de guidage à travers celle-ci.

2. Ensemble de guide-foret selon la revendication 1, dans lequel la vis (206) de blocage de pivot comprend :
une première partie cylindrique (208) connectée à une partie filetée (210), et
une deuxième partie cylindrique (212A) positionnée entre la première partie cylindrique et la partie filetée.

3. Ensemble de guide-foret selon la revendication 2,
dans lequel la première partie cylindrique (208) a un premier diamètre et la deuxième partie cylindrique (212A) a un deuxième diamètre dans lequel le premier diamètre est plus grand que le deuxième diamètre, et
dans lequel la partie filetée (210) a un troisième diamètre dans lequel le troisième diamètre est plus petit que le deuxième diamètre.

4. Ensemble de guide-foret selon la revendication 3,
dans lequel la première extrémité (202) du bras (200) de guidage comprend une extrémité fourchue avec un premier bras à extrémité fourchue et un deuxième bras à extrémité fourchue formant un premier espace entre ceux-ci,
dans lequel la première extrémité (322) du bras (320) de sonde est positionnée à l'intérieur du premier espace, et
dans lequel la vis (206) de blocage de pivot est positionnée à travers le premier bras à extrémité fourchue, le premier espace et le deuxième bras à extrémité fourchue, et dans lequel la vis de blocage de pivot est configurée pour comprimer le bras de sonde positionné à l'intérieur du premier espace et entre le premier bras à extrémité fourchue et le deuxième bras à extrémité fourchue.

5. Ensemble de guide-foret selon la revendication 1, comprenant en outre un guide-foret (408) s'étendant à travers la deuxième extrémité (406) du bras incurvé (402) d'extension de guidage.

6. Ensemble de guide-foret selon la revendication 5, comprenant en outre une sonde (302) fixée à la deuxième extrémité (324) du bras (320) de sonde ;
dans lequel la sonde est canulée et configurée pour recevoir un fil-guide (318) à travers celle-ci.

7. Ensemble de guide-foret selon la revendication 1 ou 2, dans lequel le bras (320) de sonde est rotatif jusqu'à et incluant au moins 240 degrés.

8. Ensemble de guide-foret selon la revendication 7, dans lequel la sonde (302) s'étend à travers le bras (320) de sonde.

9. Ensemble de guide-foret selon la revendication 8, dans lequel la sonde et le guide-foret (408) convergent au niveau d'un espace (102) de forage.
